# EUROPEAN PATENT SPECIFICATION

(11) **EP 2 950 776 B1**
(45) Date of publication and mention of the grant of the patent: **30.10.2019**
(21) Application number: 13808050.2
(22) Date of filing: 18.12.2013
(51) Int. Cl.: A61K 8/34, A61K 8/19, A61K 8/49, A61Q 11/00, A61K 8/81

(54) **ORAL CARE COMPOSITIONS**
MUNDPFLEGEZUSAMMENSETZUNGEN
COMPOSITIONS DE SOIN BUCCAL

(30) Priority: 01.02.2013 EP 13153689
(43) Date of publication of application: 09.12.2015
(73) Proprietor: Unilever PLC, London, Greater London EC4Y 0DY (GB); Unilever N.V., 3013 AL Rotterdam (NL)
(72) Inventor: PARKER, Andrew, Philip, Bebington Wirral Merseyside CH63 3JW (GB)
(74) Representative: Tansley, Sally Elizabeth
(86) International application number: PCT/EP2013/077209
(87) International publication number: WO 2014/117904

(56) References cited:
- WO-A1-2012/016908
- WO-A2-2012/123241
- US-A- 5 690 911
- US-A1- 2012 020 900
- US-A1- 2012 076 278

## Description

### Field of the Invention

The present invention is concerned with oral care compositions. More particularly, the present invention is concerned with oral care compositions containing a particulate tooth surface whitening agent.

### Background of the Invention

The colour of the teeth is influenced by a combination of their intrinsic colour and the presence of any extrinsic stains that may form on the tooth surface. Extrinsic colour is linked with the adsorption of materials into the acquired pellicle on the surface of enamel, which ultimately cause staining. Factors that influence extrinsic stain formation include poor tooth brushing technique, smoking, dietary intake of coloured foods (e.g. red wine), subject age and the use of certain cationic agents such as chlorhexidine or metal salts like tin and iron.

Consumers have always had a strong desire for white teeth and many individuals are dissatisfied with their current tooth colour. This desire for whiter teeth has given rise to a growing trend in the increased use of tooth whitening products.

Current whitening toothpastes rely on optimised abrasive and chemical components to maximise stain removal and prevention. During brushing, abrasive particles become temporarily trapped between the toothbrush and the stained tooth surface and abrade away the stain. Chemical components may also be used, usually in conjunction with abrasive particles, and include calcium chelators, polymers, surfactants, enzymes and oxidising agents.

Teeth whitening systems comprising SLS surfactant are disclosed in WO2012/076278, WO2012/016908, and WO2012/12324

EP 1 935 395 describes a novel optical approach to tooth whitening. On brushing with the toothpaste described in this publication, a blue pigment (in particular blue covarine) is deposited onto the tooth surface, where it is able to change the optical effects of the tooth surface, and enhance the measurement and perception of tooth whiteness. This toothpaste is intended to produce a temporary tooth whitening effect that can be reapplied as frequently as desired, as it contains no harsh chemicals, but is not intended to produce any permanent changes to the colour of the teeth. Deposition of the blue pigment onto the tooth surface is enhanced by the use of a deposition aid which is most preferably a GANTREZ® type polymer, i.e. a co-polymer of maleic anhydride with methyl vinyl ether. The toothpaste formulations described in EP 1 935 395 include an alkali-metal alkyl sulphate surfactant which is most preferably sodium lauryl sulphate (SLS). SLS is the most commonly used anionic surfactant in toothpaste formulations, with a typical toothpaste containing up to 2 or 3% of SLS (by weight based on total weight) for its foaming and surfactant action.

The present inventors have now found that the temporary tooth whitening effects delivered by a system such as that described in EP 1 935 395 can be enhanced by substituting at least part of the SLS with a different type of surfactant.

### Summary of the Invention

The present invention provides an oral care composition suitable for delivering a temporary whitening effect to the surface of teeth, the composition comprising:
(a) a liquid continuous phase comprising water or monohydric or polyhydric alcohol or a mixture thereof;
(b) a particulate tooth surface whitening agent dispersed in the continuous phase in which the particulate tooth surface whitening agent is a phthalocyanine blue pigment;
(c) a deposition aid for the particulate tooth surface whitening agent;
(d) from 0.1 to 5% by weight (based on the total weight of the oral care composition) of one or more surfactants which comprise from 0.2 to 1.0% by weight (based on the total weight of the oral care composition) of amphoteric surfactant and from 0 to 0.1% (based on the total weight of the oral care composition) of alkali-metal alkyl sulphate surfactants selected from sodium, magnesium, ammonium or ethanolamine salts of C₈ to C₁₈ alkyl sulphates and mixtures thereof and at least 50% by weight of the total surfactants present in the composition are selected from nonionic surfactants, amphoteric surfactant or zwitterionic surfactants, calcium tolerant anionic surfactants and mixtures thereof.

### Detailed Description of the Invention

### Tooth Surface Whitening Agent

The composition of the invention comprises a particulate tooth surface whitening agent which is dispersed in the continuous phase of the composition.

The particulate tooth surface whitening agent functions by being deposited onto the teeth and thereby changing their appearance. The deposition of the particulate tooth surface whitening agent onto the teeth is aided by the deposition aid. Any particulate tooth whitening agent that functions in the above manner may be used.

Typical particulate tooth surface whitening agents for use in the invention are water-insoluble solids. The term "water-insoluble" in the context of the present invention generally means an aqueous solubility of less than 10g/L at 25°C, most preferably less than 1g/L at 25°C (where the solubility is determined in un-buffered distilled water).

The colour to which the tooth surfaces are whitened depends on the colour of the particulate tooth surface whitening agent, which may for example be white, or more preferably blue. Blue particulate tooth surface whitening agents give the teeth the appearance of increased whiteness by decreasing the "yellowness" (b*) of the teeth.

Examples of white particulate tooth surface whitening agents include inorganic white pigments such as titanium dioxide, zinc oxide and zirconium oxide.

The composition comprises alpha copper phthalocyanines as
blue particulate tooth surface whitening agents for use in the invention which are designated as C.I. Pigment Blue 15, C.I. Pigment Blue 15:1 or C.I. Pigment Blue 15:2 respectively, as described in the above Table. A commercially available example is Covarine Blue W 6795, ex Sensient Cosmetic Technologies/LCW.

Further uitable blue particulate tooth surface whitening agents include blue lakes and blue pigments. A "blue" lake or pigment in the context of the present invention generally means a lake or pigment having a hue angle in the CIELAB colour space ranging from 180 to 320 degrees, preferably from 180 to 290 degrees.

Blue lakes are formed by fixing a blue dye onto a particulate inorganic substrate.

Particularly preferred blue dyes used to form the blue lake are water soluble. The term "water-soluble" in the context of the present invention generally means that the blue dye has an aqueous solubility of at least 10g/L at 25°C, most preferably at least 100g/L at 25°C (where the solubility is determined in un-buffered distilled water).

Aqueous solutions of particularly preferred blue dyes used to form the blue lake have a maximum absorbance value in the visible spectrum (λₘₐₓ) at a wavelength ranging from 550 to 650, more preferably from 600 to 650 nm.

Specific examples of preferred blue dyes used to form the blue lake include triarylmethane dyes, more preferably anionic triphenylmethane dyes, and especially diaminotriphenylmethane dyes containing from two to four sulphonate groups, such as those corresponding to general formula (I): 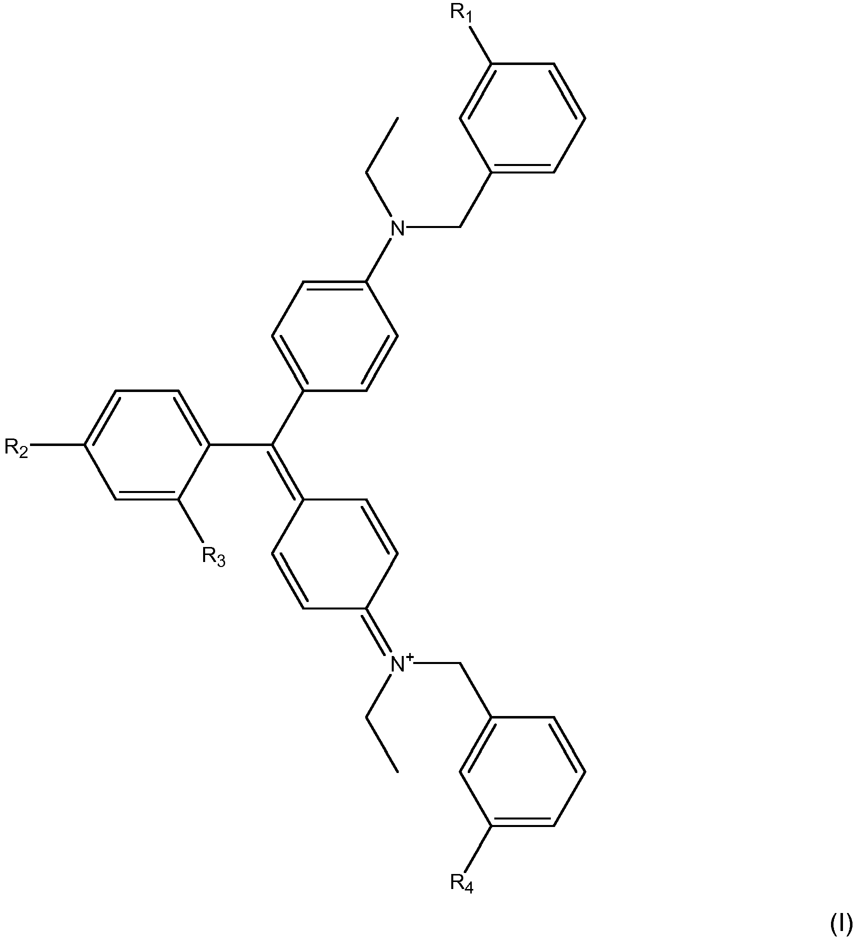 in which R¹ R² ,R³ and R⁴ are monovalent radicals which are each independently selected from hydrogen (-H), hydroxyl (-OH), halo (e.g. -Cl) and sulphonate (-SO₃⁻) groups, with the proviso that at least two of R¹ to R⁴ are sulphonate groups.

In a preferred example of a blue dye corresponding to general formula (I), R² is-H and R¹, R³, and R⁴ are sulphonate groups.

The particulate inorganic substrate onto which the blue dye is fixed is usually a colourless, inert metallic salt of synthetic or mineral origin. Examples of such materials include calcium carbonate, barium sulphate, kaolin (hydrated aluminium silicate), talc (hydrated magnesium silicate) and metal oxides such as titanium dioxide or zinc oxide. For optimum transparency the most preferred substrate is hydrated alumina, also known as alumina trihydrate or aluminium trihydroxide (Al(OH)₃). In a typical dye laking process, an aluminium salt is dissolved in water and an alkali is added to the solution in order to precipitate hydrated alumina. An aqueous solution of the blue dye is then added to a slurry of the precipitated particles, and a laking reagent (usually aluminium chloride) is added. This causes the blue dye to precipitate and adsorb onto the hydrated alumina particles, so forming the blue lake.

Typically the blue dye constitutes about 10 to 40% of the blue lake (by total weight blue dye based on the total weight of the blue lake).

Mixtures of any of the above described materials may also be used.

The most preferred blue lake for use in the invention is formed by fixing a dye of formula (I) in which R² is -H and R¹, R³, and R⁴ are sulphonate groups onto particles of hydrated alumina. This material is commercially available for example as FD&C Blue No.1 Aluminium Lake (C.I. 42090:2). The C.I. Constitution Number is taken from the Colour Index International, Fourth Edition Online (*www.colour-index.org*).

Examples of suitable blue pigments include inorganic blue pigments such as iron blue (C.I. 77510) and ultramarine blue (C.I. 77007). A preferred class of blue pigments for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contain a central, co-ordinated metal ion such as copper. Copper phthalocyanines have the basic structure:

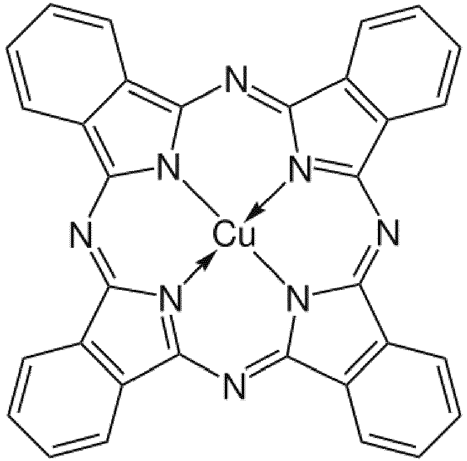

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule.

The following pigments are illustrative phthalocyanine blue pigments:

| C.I. Generic Name | C.I. Constitution Number | Crystal modification; Type | Number of halogen atoms |
|---|---|---|---|
| Pigment Blue 15 | 74 160 | alpha | - |
| Pigment Blue 15:1 | 74 160 | alpha; phase stabilised | 0.5-1 Cl |
| Pigment Blue 15:2 | 74 160 | alpha; phase & flocculation stabilised | 0.5-1 Cl |
| Pigment Blue 15:3 | 74 160 | beta | - |
| Pigment Blue 15:4 | 74 160 | beta; flocculation stabilised | - |
| Pigment Blue 15:6 | 74 160 | epsilon | - |
| Pigment Blue 16 | 74 100 | gamma; metal-free | - |

Mixtures of any of the above described materials may also be used.

The amount of particulate tooth surface whitening agent (as defined above) in compositions of the invention suitably ranges from 0.001 to 3%, preferably from 0.001 to 1%, more preferably from 0.005 to 0.3% by total weight particulate tooth surface whitening agent (as defined above) based on the total weight of the composition.

### Deposition Aid

The composition of the invention comprises a deposition aid for the particulate tooth surface whitening agent.

The term "deposition aid" in the context of this invention generally means a material which aids deposition of the tooth whitening agent from the continuous phase of the composition onto the surface of teeth during use of the composition. Use of the composition in the context of this invention typically involves application of the composition to the oral cavity, followed by brushing and/or rinsing).

Suitable deposition aids work by having affinity for both the particulate tooth surface whitening agent (as defined above) and the surface of the teeth.

Preferred deposition aids are able to aid the deposition of the particulate tooth surface whitening agent onto the teeth such that tooth surface whiteness is enhanced by at least 20% and more preferably by at least 100%, in comparison to the value obtained for teeth treated in an equivalent manner with a control formulation using the same amount of particulate tooth surface whitening agent in the absence of the deposition aid.

A convenient measure of enhanced tooth surface whiteness is delta b* measured using a chromameter. A negative value of delta b* indicates a yellow to blue colour shift which has been shown to be one of the primary drivers of tooth surface whiteness as perceived by the consumer.

Accordingly, preferred deposition aids are able to aid the deposition of the particulate tooth surface whitening agent onto the teeth such that the negative value of delta b* is augmented by at least 20% and more preferably by at least 100%, in comparison to the value obtained for teeth treated in an equivalent manner with a control formulation using the same amount of particulate tooth surface whitening agent in the absence of the deposition aid.

A simple empirical model for establishing the effect of the deposition aid is as follows:
Polished hydroxyapatite discs are first placed in sterile human saliva for 2 hours to allow a pellicle to form. The discs are then rinsed in water and baseline colour measurements made (using, for example, a Minolta chromameter CR300). The discs are then brushed with either (a) a suspension of the particulate tooth surface whitening agent in water; or (b) a suspension of the particulate tooth surface whitening agent in water at the same concentration as in (a), together with the deposition aid. The brushing is best performed using a brushing machine. Following rinsing, the colour of the discs is then re-measured and the change in delta b* is recorded for both treatment (a) and treatment (b). From a comparison of these data, the effect of the deposition aid is readily seen.

Suitable deposition aids for use in the invention will generally dissolve or disperse in water at a temperature of 25°C.

Preferred deposition aids for use in the invention are water soluble. The term "water-soluble" in this particular context generally means that the deposition aid has an aqueous solubility of at least 10g/L at 25°C, and more preferably at least 30g/L at 25°C (where the solubility is determined in un-buffered distilled water). It is particularly preferable that the deposition aid remains water soluble after drying, so that it can be re-dissolved. This prevents undesirable build up of the deposition aid on the teeth after repeated usage of the composition.

Suitable deposition aids for use in the invention include polymeric materials, preferably polymeric materials which are water soluble as defined above.

Polymeric materials for use as deposition aids in the invention may be naturally or synthetically-derived. Preferably such polymeric materials are high molecular weight. The term "high molecular weight" in this particular context generally means that the polymeric material has a molecular weight of at least 50,000, more preferably at least 500,000 g/mol. A suitable method to determine the molecular weight of such polymeric materials is gel permeation chromatography against a polyethylene glycol standard.

A preferred class of polymeric material for use as deposition aids in the invention includes water-soluble, high molecular weight polymers having anionic side groups along the polymer main chain.

Specific examples of such materials include poly(carboxylic acid) polymers. Poly (carboxylic acid) polymers are typically polymers which include -COOH groups in their structure, or groups which are derived from -COOH groups such as salt, ester or anhydride groups.

For example, the poly(carboxylic acid) polymers may include:

-[C(R¹)(COOH)-]-

units in their structure, in which R¹ is selected from hydrogen, C₁₋₃ alkyl, C₁₋₃ alkoxy or C₁₋₃ hydroxyalkyl. Preferably R¹ is hydrogen.

A preferred type of poly (carboxylic acid) polymer includes adjacent:

-[C(R¹)(COOH)-]-

units in its structure (where R¹ is as defined above), for example polymers based on maleic acid, which typically include:

-{-CH(COOH)-CH(COOH)-]-

units, and/or salts or esters of such units, or such units in anhydride form in which -COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

-[-C(R¹)(R²)-C(R³)(R⁴)-C(R⁵)(COOH)- C(R⁶)(COOH)-]-

units in its structure (and/or salts or esters of such units, or such units in anhydride form in which -COOH groups on adjacent carbon atoms are cyclised to form a ring system); in which R¹,R²,R³,R⁴,R⁵ and R⁶ are each independently selected from hydrogen, C₁₋₃ alkyl or C₁₋₃ alkoxy. Preferably R¹ and R² are hydrogen, R³ is hydrogen and R⁴ is methoxy and R⁵ and R⁶ are hydrogen.

Such a poly(carboxylic acid) polymer may be described as the polymer based on a copolymer of methyl vinyl ether and maleic anhydride, and is commercially available for example under the trade name GANTREZ®.

A particularly preferred example of such a polymer comprises:

-[-CH₂-CH(OCH₃)-CH(COOH)-CH(COOH)-]-

units in its structure, in which the -COOH groups are in free acid form. Such polymers may be linear or cross-linked. More preferably the polymer is linear. Polymers of this type are commercially available for example under the trade name GANTREZ® S. Most preferably such a polymer has a molecular weight of at least 500,000 g/mol (e.g. GANTREZ® S-96), ideally at least 1,000,000 g/mol (e.g. GANTREZ® S-97).

Alternative polymers which may be used comprise the units as described above in anhydride form, i.e. in which the two adjacent -COOH groups are cyclised to form a ring system. Such polymers are commercially available under the trade name GANTREZ® AN, e.g. GANTREZ® AN-119, GANTREZ® AN-903, GANTREZ® AN-139 and GANTREZ® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the trade name GANTREZ® MS, e.g. GANTREZ® MS-955.

Other alternative polymers which may be used comprise the units as described above in partial ester form, for example in which some of the free -COOH groups are esterified with C₁₋₆ alkyl, e.g. ethyl or n-butyl. Such polymers are commercially available under the trade name GANTREZ® ES, e.g. GANTREZ® ES-225 or GANTREZ® ES-425.

Mixtures of any of the above described materials may also be used.

The amount of deposition aid (as defined above) in compositions of the invention suitably ranges from 0.001 to 5.0%, preferably from 0.005 to 4.0%, more preferably from 0.01 to 2.0% by total weight deposition aid (as defined above) based on the total weight of the composition.

### Surfactant

The composition of the invention comprises from 0.1 to 5% by weight (based on the total weight of the oral care composition) of one or more surfactants.

The total weight of surfactant included in the composition of the invention preferably ranges from 0.2 to 1.0% by weight (based on the total weight of the oral care composition).

Of the total surfactant present in the composition, at least 50% by weight is selected from nonionic surfactants, amphoteric or zwitterionic surfactants, calcium-tolerant anionic surfactants and mixtures thereof. Preferably at least 75%, more preferably at least 80% by weight of the total surfactant present in the composition is selected from these types of surfactant.

Examples of suitable nonionic surfactants for use in the invention include condensation products of aliphatic (C8 - C18) primary or secondary linear or branched chain alcohols or phenols with alkylene oxides, usually ethylene oxide and generally having from 6 to 30 ethylene oxide groups.

Other suitable nonionic surfactants include mono- or dialkyl alkanolamides such as coconut mono- or diethanolamide and coconut monoisopropanolamide.

Other suitable nonionic surfactants include sugar-derived nonionic surfactants such as alkyl polyglycosides. Typical alkyl polyglycosides for use in the invention may be represented by the general formula RO-(G)n, wherein R is a C6 to C20 (preferably C8 to C12) alkyl group which may be saturated or unsaturated, G is a saccharide group which is preferably glucose and n represents the degree of polymerisation which is preferably from about 1.1 to about 2.

Examples of suitable amphoteric or zwitterionic surfactants for use in the invention include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, in which the alkyl and acyl groups have from 8 to 19 carbon atoms.

Specific examples of such amphoteric or zwitterionic surfactants include lauramine oxide, cocodimethyl sulphopropyl betaine, lauryl betaine, cocamidopropyl betaine and sodium cocoamphoacetate.

The calcium ion stability of anionic surfactants can be measured by the modified Hart method (Witkes, et al. J. Ind. Encl. Chem. 29,1234-1239 (1937)), carried in microtitre plates. The surfactant solution is titrated with a calcium ion solution. The onset of turbidity indicates the start of formation of insoluble calcium precipitates after a minute of shaking at room temperature.

As referred to herein, a "calcium-tolerant" anionic surfactant is one that does not precipitate at a surfactant concentration of 0.4 g/L (and at a ionic strength of a 0.040 M 1: 1 salt solution at) with a calcium concentration up to 20 FH (French hardness degrees), i.e. 200 ppm calcium.

Examples of calcium-tolerant anionic surfactants include olefin sulphonates, e.g. of C₁₀ to C₂₄ olefins, alkane and/or hydroxyalkane sulphonates, e.g. C₁₀ to C₂₄, alkyl phenoxy ether sulphates, e.g. with a C₈ to C₁₂ linear alkyl carbon chain and 1 to 10 ethylene oxide units, alkyl ether sulphates, e.g. with a C₁₀ to C₂₀ alkyl chain and 1 to 10, preferably 2 to 4 ethylene oxide groups. Other calcium-tolerant anionic surfactants include sulphocarboxylates, alkyl glyceryl ether sulphonates, monoglyceride sulphates and sulphonates and phosphated ethylene oxide-based anionic surfactants.

Mixtures of any of the above described materials may also be used.

The composition of the invention may include other types of surfactant, for example alkali-metal alkyl sulphate surfactants selected from sodium, magnesium, ammonium or ethanolamine salts of C₈ to C₁₈ alkyl sulphates and mixtures thereof.

However it is preferred that such materials (if included at all) are present in minor quantities only. For example, a composition of the invention will preferably include no more than 0.2%, more preferably no more than 0.1% by weight (based on the total weight of the oral care composition) of alkali-metal alkyl sulphate surfactants selected from sodium, magnesium, ammonium or ethanolamine salts of C₈ to C₁₈ alkyl sulphates and mixtures thereof.

A preferred composition according to the invention comprises from 0.2 to 1.0% by weight (based on the total weight of the oral care composition) of nonionic surfactant and from 0 to 0.1% (based on the total weight of the oral care composition) of alkali-metal alkyl sulphate surfactants selected from sodium, magnesium, ammonium or ethanolamine salts of C₈ to C₁₈ alkyl sulphates and mixtures thereof. Most preferably the nonionic surfactant in such a composition is an alkyl polyglycoside of general formula RO-(G)n, wherein R is a C₆ to C₂₀ (preferably C₈ to C₁₂) alkyl group which may be saturated or unsaturated, G is a saccharide group which is preferably glucose and n represents the degree of polymerisation which is preferably from about 1.1 to about 2.

Thecomposition according to the invention comprises from 0.2 to 1.0% by weight (based on the total weight of the oral care composition) of amphoteric surfactant, and from 0 to 0.1% (based on the total weight of the oral care composition) of alkali-metal alkyl sulphate surfactants selected from sodium, magnesium, ammonium or ethanolamine salts of C₈ to C₁₈ alkyl sulphates and mixtures thereof. Most preferably the amphoteric surfactant in such a composition is an alkyl amine oxide or an alkyl amidopropyl betaine, in which the alkyl groups have from 8 to 19 carbon atoms.

### Product Form

The composition of the invention comprises a liquid continuous phase comprising water or monohydric or polyhydric alcohol or a mixture thereof.

Preferred product forms for compositions of the invention are those which are suitable for rinsing the surfaces of the oral cavity.

In such preferred product forms, the amount of water will generally be at least 15wt%, preferably at least 30wt%, more preferably at least 50wt% (by weight based on the total weight of the composition).

A preferred type of product form in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition according to the invention will usually contain, as the liquid continuous phase, a mixture of water and polyhydric alcohol in various relative amounts, with the amount of water generally ranging from 70 to 90wt% (by weight based on the total weight of the mouthwash), and the amount of polyhydric alcohol generally ranging from 5 to 20wt% (by weight based on the total weight of the mouthwash).

Typical polyhydric alcohols for use in a mouthwash composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A mouthwash composition suitable for use in the invention may also contain a monohydric alcohol such as ethanol, isopropanol or a mixture thereof. If present, the amount of monohydric alcohol typically ranges from 1 to 25%, preferably from 10 to 20% by weight based on the total weight of the mouthwash.

Mouthwash compositions may be provided in a "ready to use" form; as a concentrated solution, for dilution by the user immediately prior to use; or in solid form, such as a tablet or in a sachet, for dissolution by the user immediately prior to use. Tablets may suitably be prepared using xylitol and/or sorbitol as the major ingredient. The sachets and tablets may be formulated to provide, on dissolution, a still mouthwash, or, by the incorporation of a suitable effervescent couple, for instance sodium carbonate/bicarbonate and citric acid, an effervescent mouthwash.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

The invention is further illustrated with reference to the following, non-limiting Examples.

### EXAMPLES

Mouthwash formulations were prepared having the ingredients shown in Table 1 below, in which all ingredients are expressed by weight percent of the total formulation.

Examples 1 to 3 represent formulations according to the invention. Example A is a comparative example (not according to the invention).

**Table 1**

| **Ingredient** | **Ex.A** | **Ex.1** | **Ex.2** | **Ex.3** |
|---|---|---|---|---|
| Water | 83.155 | 82.365 | 82.815 | 82.365 |
| Sorbitol (70% a.i.) | 12.00 | 12.00 | 12.00 | 12.00 |
| Trisodium phosphate | 0.60 | 0.60 | 0.60 | 0.60 |
| PEG40 hydrogenated castor oil | 2.50 | 2.50 | 2.50 | 2.50 |
| GANTREZ® S-97 | 0.50 | 0.50 | 0.50 | 0.50 |
| Sodium lauryl sulphate | 0.34 | - | - | - |
| EMPIGEN® OB (lauramine oxide, 30% a.i,. ex Huntsman) | - | 1.13 | - | - |
| GLUCOPON® 600 CS UP (lauryl glucoside, 50% a.i., ex Cognis) | - | - | 0.68 | - |
| EMPIGEN® BS/FA (cocamidopropylbetaine, 30% a.i., ex Huntsman) | - | - | - | 1.13 |
| Phenoxyethanol | 0.30 | 0.30 | 0.30 | 0.30 |
| Benzyl alcohol | 0.30 | 0.30 | 0.30 | 0.30 |
| Blue Covarine W6795 (C.I.74160), 40% a.i. | 0.005 | 0.005 | 0.005 | 0.005 |
| Flavour | 0.30 | 0.30 | 0.30 | 0.30 |

The above formulations were evaluated for their ability to deliver an optical whitening effect to the surface of teeth, as follows:

### Experimental Method

A random selection of 32 extracted human teeth was taken and cleaned using a commercially available toothpaste (Signal Integral™). After thorough rinsing with demineralised water, the teeth were placed in irradiated human saliva in order to generate a pellicle layer on the surface.

100ml of the test formulation was placed in an evaporating basin on a magnetic stirrer. Two teeth were taken at random and the colour (CIELAB coordinates) measured using a chromometer (Minolta Chroma Meter CR-321) relative to a white calibration tile. The teeth were then immersed in the test formulation for 60 seconds. Excess formulation was then removed by gently shaking the teeth. The colour was then remeasured. This was repeated using 8 teeth for each formulation.

### Results

For each set of teeth, the average L*, a* and b* values were calculated for before and after application. The difference (dL*, da* and db*) were then calculated. The results are shown in Table 2 below.

**Table 2**

| **Average** | | | |
|---|---|---|---|
| | **dL*** | **da*** | **db*** |
| Example A | -0.23 | -0.11 | -0.36 |
| Example 1 | -0.69 | -0.27 | -0.84 |
| Example 2 | -0.54 | -0.23 | -0.77 |
| Example 3 | -0.62 | -0.15 | -0.79 |

| **95% Confidence limits** | | | |
|---|---|---|---|
| | **dL*** | **da*** | **db*** |
| Example A | 0.21 | 0.12 | 0.15 |
| Example 1 | 0.24 | 0.09 | 0.21 |
| Example 2 | 0.34 | 0.10 | 0.06 |
| Example 3 | 0.31 | 0.17 | 0.08 |

The results clearly show that Examples 1 to 3 (according to the invention) deliver a larger change in b* compared to Example A (not according to the invention). This provides a superior whitening effect by countering the natural yellowness of the teeth.

## Claims

1. An oral care composition suitable for delivering a temporary whitening effect to the surface of teeth, the composition comprising:
(a) a liquid continuous phase comprising water or monohydric or polyhydric alcohol or a mixture thereof;
(b) a particulate tooth surface whitening agent dispersed in the continuous phase in which the particulate tooth surface whitening agent is a phthalocyanine blue pigment; ,
(c) a deposition aid for the particulate tooth surface whitening agent;
(d) from 0.1 to 5% by weight (based on the total weight of the oral care composition) of one or more surfactants which comprise from 0.2 to 1.0% by weight (based on the total weight of the oral care composition) of amphoteric surfactant and from 0 to 0.1 % (based on the total weight of the oral care composition) of alkali-metal alkyl sulphate surfactants selected from sodium, magnesium, ammonium or ethanolamine salts of C₈ to C₁₈ alkyl sulphates and mixtures thereof;
and at least 50% by weight of the total surfactants present in the composition are selected from nonionic surfactants, amphoteric surfactant or zwitterionic surfactants, calcium tolerant anionic surfactants and mixtures thereof.

2. An oral care composition according to claim 1, in which of the total surfactants (d) present in the composition, at least 80% by weight are selected from nonionic surfactants, amphoteric or zwitterionic surfactants, calcium-tolerant anionic surfactants and mixtures thereof.

3. An oral care composition according to any preceding claim, in which the phthalocyanine blue pigment is selected from those alpha copper phthalocyanines which are designated as C.I.Pigment Blue 15, C.I. Pigment Blue 15:1 or C.I. Pigment Blue 15:2.

4. An oral care composition according to any preceding claim, in which the deposition aid is a poly(carboxylic acid) polymer having a molecular weight of at least 50,000 g/mol and an aqueous solubility of at least 10g/L at 25°C.

5. An oral care composition according to any preceding claim, which is in the form of a mouthwash containing, as the liquid continuous phase, a mixture of water and polyhydric alcohol, with the amount of water ranging from 70 to 90wt% (by weight based on the total weight of the mouthwash), and the amount of polyhydric alcohol ranging from 5 to 20wt% (by weight based on the total weight of the mouthwash).

## Patentansprüche

1. Mundpflegezusammensetzung, die geeignet ist, der Oberfläche von Zähnen einen vorübergehenden Eindruck der Aufhellung zu geben, wobei die Zusammensetzung umfasst:
(a) eine flüssige kontinuierliche Phase, umfassend Wasser oder einwertigen oder mehrwertigen Alkohol oder eine Mischung davon;
(b) ein teilchenförmiges Aufhellungsmittel für die Zahnoberfläche, dispergiert in der kontinuierlichen Phase, in der das teilchenförmige Aufhellungsmittel für die Zahnoberfläche ein Phthalocyaninblau-Pigment ist;
(c) ein Ablagerungshilfsmittel für das teilchenförmige Aufhellungsmittel für die Zahnoberfläche;
(d) von 0,1 bis 5 Gewichts-% (bezogen auf das Gesamtgewicht der Mundpflegezusammensetzung) eines oder mehrerer Tenside, die von 0,2 bis 1,0 Gewichts-% (bezogen auf das Gesamtgewicht der Mundpflegezusammensetzung) an amphoterem Tensid und von 0 bis 0,1% (bezogen auf das Gesamtgewicht der Mundpflegezusammensetzung) an Alkalimetallalkylsulfat-Tensiden, ausgewählt aus Natrium-, Magnesium-, Ammonium- oder Ethanolamin-Salzen von C₈- bis C₁₈-Alkylsulfaten und Mischungen davon, umfasst;
und mindestens 50 Gewichts-% der gesamten Tenside, die in der Zusammensetzung vorliegen, unter nichtionischen Tensiden, amphoterem Tensid oder zwitterionischen Tensiden, Calcium-toleranten anionischen Tensiden und Mischungen davon ausgewählt sind.

2. Mundpflegezusammensetzung nach Anspruch 1, in der von den gesamten Tensiden (d), die in der Zusammensetzung vorliegen, mindestens 80 Gewichts-% unter nichtionischen Tensiden, amphoteren oder zwitterionischen Tensiden, Calcium-toleranten anionischen Tensiden und Mischungen davon ausgewählt sind.

3. Mundpflegezusammensetzung nach irgendeinem vorhergehenden Anspruch, in welcher das Phthalocyaninblau-Pigment unter denjenigen alpha-Kupferphthalocyaninen ausgewählt ist, welche als C. I. Pigment Blue 15, C. I. Pigment Blue 15:1 oder C. I. Pigment Blue 15:2 bezeichnet werden.

4. Mundpflegezusammensetzung nach irgendeinem vorhergehenden Anspruch, in welcher das Ablagerungshilfsmittel Poly(carbonsäure)-Polymer eines Molekulargewichts von mindestens 50.000 g/mol und einer Wasserlöslichkeit von mindestens 10 g/l bei 25°C ist.

5. Mundpflegezusammensetzung nach irgendeinem vorhergehenden Anspruch, welche in Form eines Mundwassers vorliegt, enthaltend als flüssige kontinuierliche Phase, eine Mischung von Wasser und mehrwertigem Alkohol, wobei die Menge des Wassers zwischen 70 und 90 Gew.-% (in Gewicht, bezogen auf das Gesamtgewicht des Mundwassers), und wobei die Menge des mehrwertigen Alkohols zwischen 5 und 20 Gew.-% (in Gewicht, bezogen auf das Gesamtgewicht des Mundwassers) liegt.

## Revendications

1. Composition pour le soin oral appropriée pour fournir un effet de blanchiment temporaire à la surface de dents, la composition comprenant :
(a) une phase continue liquide comprenant de l'eau ou un alcool monovalent ou polyvalent ou un mélange de ceux-ci ;
(b) un agent blanchissant la surface de dent particulaire dispersé dans la phase continue dans laquelle l'agent blanchissant la surface de dent particulaire est un pigment bleu de phtalocyanine ;
(c) un aide au dépôt pour l'agent blanchissant la surface de dent particulaire ;
(d) de 0,1 à 5 % en masse (rapporté à la masse totale de la composition pour le soin oral) d'un ou plusieurs tensioactifs qui comprennent de 0,2 à 1,0 % en masse (rapporté à la masse totale de la composition pour le soin oral) de tensioactif amphotère et de 0 à 0,1 % (rapporté à la masse totale de la composition pour le soin oral) de tensioactifs d'alkylsulfates de métaux alcalins choisis parmi des sels de sodium, magnésium, ammonium ou éthanolamine d'alkylsulfates en C₈ à C₁₈ et des mélanges de ceux-ci ;
et au moins 50 % en masse de tous les tensioactifs présents dans la composition sont choisis parmi des tensioactifs non ioniques, tensioactifs amphotères ou tensioactifs zwitterioniques, tensioactifs anioniques tolérant le calcium et mélanges de ceux-ci.

2. Composition pour le soin oral selon la revendication 1, dans laquelle de tous les tensioactifs (d) présents dans la composition, au moins 80 % en masse sont choisis parmi des tensioactifs non ioniques, tensioactifs amphotères ou zwitterioniques, tensioactifs anioniques tolérant le calcium et mélanges de ceux-ci.

3. Composition pour le soin oral selon l'une quelconque des revendications précédentes, dans laquelle le pigment bleu de phtalocyanine est choisi parmi des phtalocyanines de cuivre alpha qui sont désignées comme pigment bleu C.I. 15, pigment bleu C.I. 15:1 ou pigment bleu C.I. 15:2.

4. Composition pour le soin oral selon l'une quelconque des revendications précédentes, dans laquelle l'aide au dépôt est un polymère de poly(acide carboxylique) présentant une masse moléculaire d'au moins 50 000 g/mol et une solubilité aqueuse d'au moins 10 g/l à 25°C.

5. Composition pour le soin oral selon l'une quelconque des revendications précédentes, qui est dans la forme d'une eau de bouche contenant, comme phase continue liquide, un mélange d'eau et d'alcool polyvalent, avec la quantité d'eau étant de 70 à 90 % en masse (en masse rapporté à la masse totale de l'eau de bouche), et la quantité d'alcool polyvalent étant de 5 à 20 % en masse (en masse rapporté à la masse totale de l'eau de bouche).
